# EUROPEAN PATENT APPLICATION

(11) **EP 4 582 810 A2**
(43) Date of publication of application: **09.07.2025**
(21) Application number: 25170324.5
(22) Date of filing: 22.12.2021
(51) Int. Cl.: G01N 33/545

(54) **METHOD FOR MEASURING TARGET ANTIGEN, AND INSOLUBLE PARTICLES AND KIT FOR TARGET ANTIGEN MEASUREMENT USED THEREIN**

(30) Priority: 25.12.2020 JP 2020217745
(62) Divisional of application: 21910898.2
(71) Applicant: Denka Company Limited, Tokyo 103-8338 (JP)
(72) Inventor: SHIMOMOTO, Masako, Tokyo 103-8338 (JP)
(74) Representative: Hoffmann Eitle

(57) **Abstract**

Insoluble particles containing a granular carrier and an antibody to a target antigen carried on the granular carrier, no sugar chain being bonded to the heavy chain of the antibody.

## Description

### Technical Field

The present invention relates to a method for measuring a target antigen, and insoluble particles and a kit for measuring a target antigen used therein.

### Background Art

As a method for measuring a target antigen using an immune response, there is a method using granular carriers such as latex (for example, Patent Literature 1). Granular carriers to which antibodies specific to a target antigen are bound cause an antigen-antibody reaction in the presence of the target antigen. Then, due to their specificity and affinity, they bind and aggregate with each other by bridging between the target antigen. The presence or absence and amount of the target antigen are measured based on the presence or absence of produced aggregates or the degree of aggregation.

Such a measurement method using an immune response may have a problem of insufficient detection sensitivity. For example, Patent Literature 2 discloses polysaccharide-modified composite particles in which the occurrence of nonspecific adsorption is reduced so that the measurement sensitivity is not reduced.

### Citation List

### Patent Literature

[Patent Literature 1] JP 2017-83440
[Patent Literature 2] JP 2009-162537

### Summary of Invention

### Technical Problem

However, the detection sensitivity described above still has room for improvement. Here, a granular carrier (contained in insoluble particles) to which antibodies specific to a target antigen are bound allowing a high degree of aggregation may improve the detection sensitivity, since it is assumed that the detection will be able to be performed even with a trace amount of the target antigen.

An object of the present invention is to provide an insoluble particle with high aggregation ability, and a kit for measuring a target antigen and a method for measuring a target antigen using the insoluble particle.

### Solution to Problem

The present inventors have found that a granular carrier carrying an antibody with no sugar chain bound to its heavy chain is more likely to aggregate than a granular carrier carrying an antibody with a sugar chain bound to its heavy chain.

The present invention relates to an insoluble particle comprising: a granular carrier; and an antibody against a target antigen carried on the granular carrier wherein no sugar chain is bound to a heavy chain of the antibody.

The present invention relates to a kit for measuring a target antigen, comprising: the insoluble particle; or an antibody or a granular carrier for preparing the insoluble particle.

The present invention also relates to a method for measuring a target antigen wherein the insoluble particle is used.

### Advantageous Effects of Invention

According to the present invention, it is possible to provide an insoluble particle having excellent aggregation ability when an antigen-antibody reaction occurs. High-sensitivity detection of a target antigen can be expected by the insoluble particle having excellent aggregation ability.

### Brief Description of Drawings

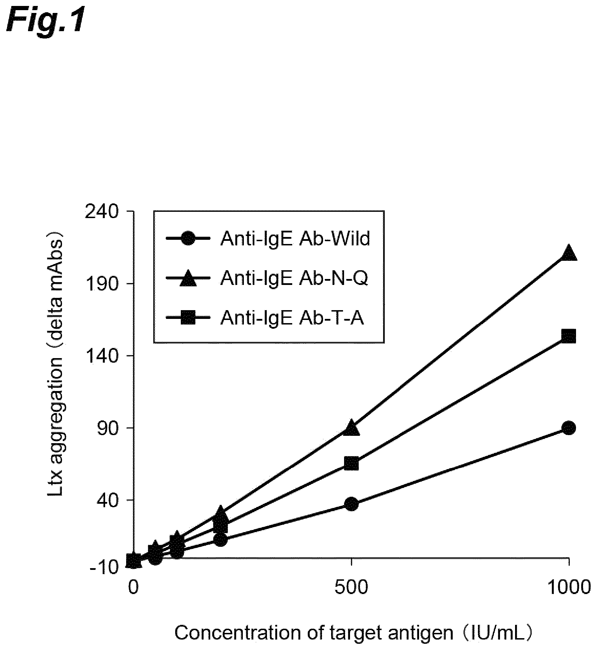

FIG. 1 is a graph showing results of aggregation reactions of anti-IgE antibody-sensitized latex particles.

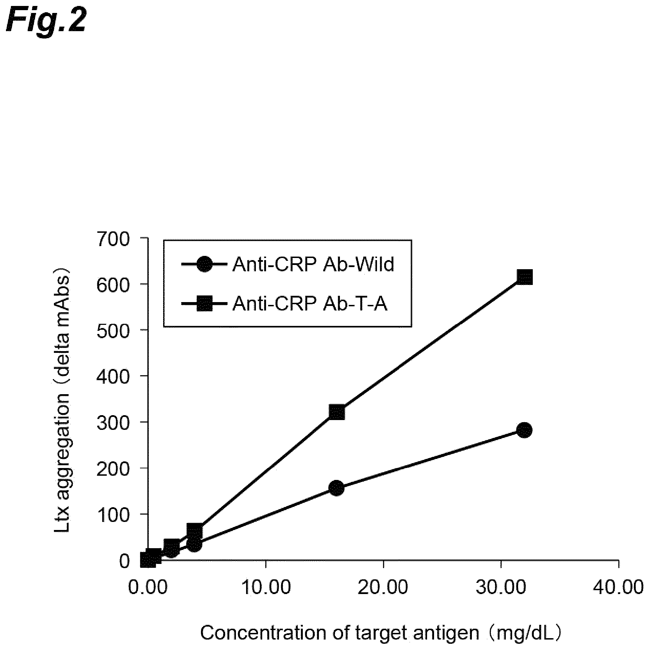

FIG. 2 is a graph showing results of aggregation reactions of anti-CRP antibody-sensitized latex particles.
FIG. 3 is a schematic diagram showing T-DNA regions of a TMV vector (plCH56122) and a PVX vector (pICH56131).

### Description of Embodiments

Hereinafter, embodiments of the present invention will be described in detail.

An insoluble particle according to one embodiment comprises: a granular carrier; and an antibody against a target antigen carried on the granular carrier wherein no sugar chain is bound to a heavy chain of the antibody. The antibody against the target antigen may be in a mode in which no sugar chain bound not only to a heavy chain, but also to a light chain.

Examples of the target antigen include protein markers such as CRP (C-reactive protein), prostate-specific antigen, ferritin, β-2-microglobulin, myoglobin, hemoglobin, albumin, and creatinine; immunoglobulins such as IgG, IgE, IgA, and IgM; various tumor markers; lipoproteins such as LDL, HDL, and TG; virus antigens such as influenza A virus, influenza B virus, RS virus (RSV), rhinovirus, rotavirus, norovirus, adenovirus, astrovirus, HAV, HBs, HCV, HIV, and EBV; bacterial antigens such as *Chlamydia trachomatis, Streptococcus pyogenes, Bordetella pertussis, Helicobacter pylori, Leptospira, Treponema pallidum, Toxoplasma gondii, Borrelia,* genus *Legionella, Bacillus anthracis,* and MRSA; toxins produced by bacteria; mycoplasma lipid antigens; peptide hormones such as human chorionic gonadotropin; steroids such as steroid hormones; bioactive amines such as epinephrine or morphine; vitamins such as vitamin Bs; prostaglandins; antibiotics such as tetracycline; pesticides, and endocrine disruptors, but are not limited thereto.

Examples of the granular carrier include latex particles, ceramic particles, alumina particles, silica-alumina particles, and carbon black particles. Latex particles are preferable among these particles. Examples of latex materials include polystyrene and divinylbenzene, and polystyrene is preferable. The average particle diameter of the granular carrier can be 0.1 to 5 µm. The particle diameters of the granular carrier can be measured through a dynamic light scattering method. The term "average particle diameter" in the present specification means a particle diameter when a cumulative value from the small particle diameter reaches 50% of the total in a volume-based particle diameter distribution curve obtained through a dynamic light scattering method.

The antibody is not particularly limited as long as it can specifically bind to the target antigen, and may be any of IgG, IgM, IgA, IgD, and IgE, but is preferably IgG. IgG is composed of two heavy chains (H chains) and two light chains (L chains). The heavy chain is composed of a variable region (VH), a first constant region (CH1), a second constant region (CH2), and a third constant region (CH3) in this order from the N-terminus. A light chain is composed of a variable region (VL) and a constant region (CL) in this order from the N-terminus. A portion composed of CH2 and CH3 is an Fc region. One heavy chain and one light chain are bound to each other through a disulfide bond between a cysteine residue present in CH1 and a cysteine residue present in CL. In addition, heavy chains are bound to each other through a disulfide bond between cysteine residues present in a hinge region located between CH1 and CH2. The antibody may be a fragment of IgG with a heavy chain, may be rIgG (reduced IgG) composed of one heavy chain and one light chain, may be a fragment composed of two heavy chains, or may be a fragment composed of one heavy chain. In an antibody with two heavy chains, at least one heavy chain may be free of a sugar chain bound thereto, or both heavy chains may be free of sugar chains bound thereto.

In a case where the antibody is IgG, an Fc region thereof preferably has an amino acid sequence having 90% or more identity with any one of amino acid sequences of SEQ ID NOs: 1 to 13, but does not have a sugar chain-binding motif Asn-Xaa-Ser/Thr (Xaa is any amino acid).

SEQ ID NO: 1 is protein accession no. P01857 (human Ig gamma-1 C region), SEQ ID NO: 2 is protein accession no. P01859 (human Ig gamma-2 C region), SEQ ID NO: 3 is protein accession no. P01860 (human Ig gamma-3 C region), SEQ ID NO: 4 is protein accession no. P01861 (human Ig gamma-4 C region), SEQ ID NO: 5 is protein accession no. P01868 (mouse Ig gamma-1 C region), SEQ ID NO: 6 is protein accession no. P01863 (mouse Ig gamma-2a C region), SEQ ID NO: 7 is protein accession no. P01867 (mouse Ig gamma-2b C region), SEQ ID NO: 8 is protein accession no. P03987 (mouse Ig gamma-3 C region), SEQ ID NO: 9 is protein accession no. P20759 (rat Ig gamma-1 C region), SEQ ID NO: 10 is protein accession no. P20760 (rat Ig gamma-2a C region), SEQ ID NO: 11 is protein accession no. P20761 (rat Ig gamma-2b C region), SEQ ID NO: 12 is protein accession no. P20762 (rat Ig gamma-2c C region), and SEQ ID NO: 13 is protein accession no. P01870 (rabbit Ig gamma C region).

In a case where the antibody is IgG, an Fc region thereof may have an amino acid sequence of SEQ ID NOs: 14 to 26 or an amino acid sequence having 90% or more identity with the amino acid sequence of SEQ ID NOs: 14 to 26 wherein Asp at position 180 of SEQ ID NO: 14, at position 176 of SEQ ID NO: 15, at position 227 of SEQ ID NO: 16, at position 177 of SEQ ID NO: 17, at position 174 of SEQ ID NO: 18, at position 180 of SEQ ID NO: 19, at position 185 of SEQ ID NO: 20, at position 179 of SEQ ID NO: 21, at position 176 of SEQ ID NO: 22, at position 172 of SEQ ID NO: 23, at position 183 of SEQ ID NO: 24, at position 179 of SEQ ID NO: 25, or at position 173 of SEQ ID NO: 26 is not mutated.

SEQ ID NO: 14 is a sequence in which Asn at position 180 of SEQ ID NO: 1 is replaced with Asp, SEQ ID NO: 15 is a sequence in which Asn at position 176 of SEQ ID NO: 2 is replaced with Asp, SEQ ID NO: 16 is a sequence in which Asn at position 227 of SEQ ID NO: 3 is replaced with Asp, SEQ ID NO: 17 is a sequence in which Asn at position 177 of SEQ ID NO: 4 is replaced with Asp, SEQ ID NO: 18 is a sequence in which Asn at position 174 of SEQ ID NO: 5 is replaced with Asp, SEQ ID NO: 19 is a sequence in which Asn at position 180 of SEQ ID NO: 6 is replaced with Asp, SEQ ID NO: 20 is a sequence in which Asn at position 185 of SEQ ID NO: 7 is replaced with Asp, SEQ ID NO: 21 is a sequence in which Asn at position 179 of SEQ ID NO: 8 is replaced with Asp in SEQ ID NO: 21, SEQ ID NO: 22 is a sequence in which Asn at position 176 of SEQ ID NO: 9 is replaced with Asp, SEQ ID NO: 23 is a sequence in which Asn at position 172 of SEQ ID NO: 10 is replaced with Asp, SEQ ID NO: 24 is a sequence in which Asn at position 183 of SEQ ID NO: 11 is replaced with Asp, SEQ ID NO: 25 is a sequence in which Asn at position 179 of SEQ ID NO: 12 is replaced with Asp, and SEQ ID NO: 26 is a sequence in which Asn at position 173 of SEQ ID NO: 13 is replaced with Asp.

In a case where the antibody is IgG, an Fc region thereof may have an amino acid sequence of SEQ ID NOs: 27 to 39 or an amino acid sequence having 90% or more identity with the amino acid sequence of SEQ ID NOs: 27 to 39 wherein Gln at position 180 of SEQ ID NO: 27, at position 176 of SEQ ID NO: 28, at position 227 of SEQ ID NO: 29, at position 177 of SEQ ID NO: 30, at position 174 of SEQ ID NO: 31, at position 180 of SEQ ID NO: 32, at position 185 of SEQ ID NO: 33, at position 179 of SEQ ID NO: 34, at position 176 of SEQ ID NO: 35, at position 172 of SEQ ID NO: 36, at position 183 of SEQ ID NO: 37, at position 179 of SEQ ID NO: 38, or at position 173 of SEQ ID NO: 39 is not mutated.

SEQ ID NO: 27 is a sequence in which Asn at position 180 of SEQ ID NO: 1 is replaced with Gln, SEQ ID NO: 28 is a sequence in which Asn at position 176 of SEQ ID NO: 2 is replaced with Gln, SEQ ID NO: 29 is a sequence in which Asn at position 227 of SEQ ID NO: 3 is replaced with Gln, SEQ ID NO: 30 is a sequence in which Asn at position 177 of SEQ ID NO: 4 is replaced with Gln, SEQ ID NO: 31 is a sequence in which Asn at position 174 of SEQ ID NO: 5 is replaced with Gln, SEQ ID NO: 32 is a sequence in which Asn at position 180 of SEQ ID NO: 6 is replaced with Gln, SEQ ID NO: 33 is a sequence in which Asn at position 185 of SEQ ID NO: 7 is replaced with Gln, SEQ ID NO: 34 is a sequence in which Asn at position 179 of SEQ ID NO: 8 is replaced with Gln, SEQ ID NO: 35 is a sequence in which Asn at position 176 of SEQ ID NO: 9 is replaced with Gln, SEQ ID NO: 36 is a sequence in which Asn at position 172 of SEQ ID NO: 10 is replaced with Gln, SEQ ID NO: 37 is a sequence in which Asn at position 183 of SEQ ID NO: 11 is replaced with Gln, SEQ ID NO: 38 is a sequence in which Asn at position 179 of SEQ ID NO: 12 is replaced with Gln, and SEQ ID NO: 39 is a sequence in which Asn at position 173 of SEQ ID NO: 13 is replaced with Gln.

In a case where the antibody is IgG, an Fc region thereof may have an amino acid sequence of SEQ ID NOs: 40 to 52 or an amino acid sequence having 90% or more identity with the amino acid sequence of SEQ ID NOs: 40 to 52 wherein Ala at position 182 of SEQ ID NO: 40, at position 178 of SEQ ID NO: 41, at position 229 of SEQ ID NO: 42, at position 179 of SEQ ID NO: 43, at position 176 of SEQ ID NO: 44, at position 182 of SEQ ID NO: 46, at position 187 of SEQ ID NO: 45, at position 181 of SEQ ID NO: 47, at position 178 of SEQ ID NO: 48, at position 174 of SEQ ID NO: 49, at position 185 of SEQ ID NO: 50, at position 181 of SEQ ID NO: 51, or at position 175 of SEQ ID NO: 52 is not mutated.

SEQ ID NO: 40 is a sequence in which Thr at position 182 of SEQ ID NO: 1 is replaced with Ala, SEQ ID NO: 41 is a sequence in which Thr at position 178 of SEQ ID NO: 2 is replaced with Ala, SEQ ID NO: 42 is a sequence in which Thr at position 229 of SEQ ID NO: 3 is replaced with Ala, SEQ ID NO: 43 is a sequence in which Thr at position 179 of SEQ ID NO: 4 is replaced with Ala, SEQ ID NO: 44 is a sequence in which Thr at position 176 of SEQ ID NO: 5 is replaced with Ala, SEQ ID NO: 45 is a sequence in which Thr at position 182 of SEQ ID NO: 6 is replaced with Ala, SEQ ID NO: 46 is a sequence in which Thr at position 187 of SEQ ID NO: 7 is replaced with Ala, SEQ ID NO: 47 is a sequence in which Thr at position 181 of SEQ ID NO: 8 is replaced with Ala, SEQ ID NO: 48 is a sequence in which Thr at position 178 of SEQ ID NO: 9 is replaced with Ala, SEQ ID NO: 49 is a sequence in which Thr at position 174 of SEQ ID NO: 10 is replaced with Ala, SEQ ID NO: 50 is a sequence in which Thr at position 185 of SEQ ID NO: 11 is replaced with Ala, SEQ ID NO: 51 is a sequence in which Thr at position 181 of SEQ ID NO: 12 is replaced with Ala, and SEQ ID NO: 52 is a sequence in which Thr at position 175 of SEQ ID NO: 13 is replaced with Ala.

The antibody with no sugar chain bound to its heavy chain can be produced using a plant transient expression system and can also be produced using a genetically modified plant. As plant transient expression systems, a method disclosed in Japanese Patent No. 5015012 can be used, for example. In addition, the antibody with no sugar chain bound to its heavy chain can also be obtained as a recombinant in which mammalian cells are used as expression hosts. Examples of mammalian cells in that case include, but are not limited to, CHO (Chinese hamster ovary) cells and HEK293 cells (human embryonic kidney cells 293). Examples of techniques of obtaining a recombinant include a transient expression system in which a plasmid vector or a virus vector lacking autonomous replication ability is used, a semi-stable expression system in which an episomal vector that has a nuclear localization signal imparted and has autonomous replication ability is used, and a stable expression system in which a target gene is inserted into a genome of an expression host, but are not particularly limited.

General techniques, for example, a physical adsorption method and a chemical binding method, can be used to bind the granular carrier to the antibody with no sugar chain bound to its heavy chain.

In a method for measuring a target antigen of one embodiment, the insoluble particle is used. The measurement method can be performed to qualitatively evaluate the presence or absence of the target antigen in a test sample or to quantitatively evaluate a concentration of the target antigen in a test sample.

In one embodiment, the measurement method comprises: a step of bringing the insoluble particles into contact with a test sample that may contain the target antigen; and a step of measuring an aggregation reaction of the insoluble particles due to an antigen-antibody reaction of the antibody and the target antigen. As for the insoluble particles, those that have already been prepared may be used, and insoluble particles may be prepared by binding a granular carrier to an antibody with no sugar chain bound to its heavy chain upon measurement.

By mixing a suspension comprising the insoluble particles with the test sample, the insoluble particles can be brought into contact with the test sample that may contain a target antigen. When the two are brought into contact with each other, the insoluble particles are aggregated by an interaction between the target antigen contained in the test sample and the antibody carried on the granular carrier, and the absorbance of the suspension changes. The amount of change in absorbance (endpoint method) or the change rate (rate method) is measured. Turbidimetric or colorimetric methods are suitably used for the measurement. For example, light in the visible to near-infrared region, usually 300 nm to 1,000 nm, preferably 500 nm to 900 nm, is emitted from outside a cell, a change in absorbance or a change in intensity of scattered light is detected to measure the aggregation reaction of the insoluble particles.

The time for performing the aggregation reaction can be 1 minute to 30 minutes, preferably 1 minute to 10 minutes, but is not limited thereto. The temperature for performing the aggregation reaction can be 35°C to 40°C and can also be 36°C to 38°C, but is not limited thereto.

A plurality of standard samples containing various known concentrations of the target antigen to be measured are prepared, and the amount or rate of change in absorbance for each of them is measured through the method. A calibration curve is drawn by plotting the concentrations of the antigen to be measured in the standard samples on the horizontal axis and the measured amount or rate of change in absorbance on the vertical axis. The amount or rate of change in absorbance for an unknown test sample can be measured through the same method, and measurement results can be applied to the calibration curve to quantitatively evaluate the target antigen in the test sample. In addition, a threshold value for the amount or rate of change in absorbance is set in advance, and in a case where the threshold value is exceeded, the presence of the target antigen in the test sample can be qualitatively evaluated.

The test sample is not particularly limited as long as it can contain a target antigen, and examples thereof include body fluids such as blood, serum, plasma, urine, feces, saliva, tissue fluid, cerebrospinal fluid, and swab fluid, or dilutions thereof, and blood, serum, plasma, urine, feces, cerebrospinal fluid, or dilutions thereof are preferable.

A kit for measuring a target antigen according to one embodiment comprises the insoluble particles. The kit for measuring the target antigen can be used for qualitatively evaluating the presence or absence of the target antigen in a test sample or quantitatively evaluating the concentration of the target antigen in a test sample, and more specifically, it can be used in the method for measuring the target antigen.

In one embodiment, the kit for measuring the target antigen comprises a granular carrier and an antibody with no sugar chain bound to its heavy chain and may be in the form of binding the two to prepare a granular carrier that carries the antibody with no sugar chain bound to its heavy chain upon measurement.

The kit for measuring the target antigen may further comprises the target antigen for a positive control or use in creating a calibration curve, or may further comprise a buffer solution for diluting the test sample, a buffer solution for mixing the insoluble particles with the test sample, a buffer solution for binding the granular carrier to the antibody with no sugar chain bound to its heavy chain, and the like.

### [Examples]

### <Preparation of anti-IgE antibody (Anti-IgE Ab-Wild)>

An antibody whose heavy chain constant region consists of SEQ ID NO: 5 was produced through the following method.

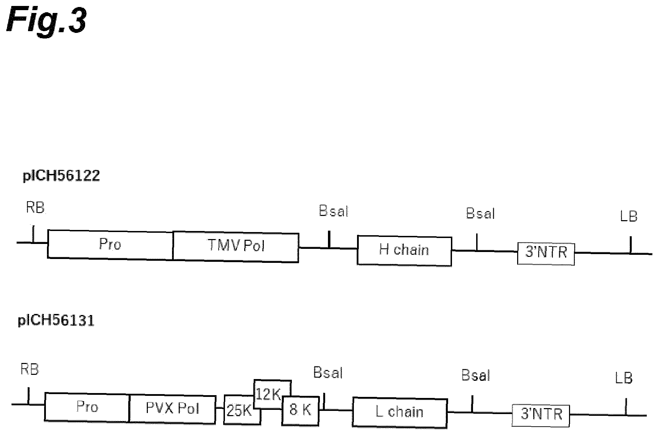

It was prepared in a plant transient expression system disclosed in Japanese Patent No. 5015012. Tobacco mosaic virus (TMV) vector pICH56122 (FIG. 3) and a synthetic DNA containing an anti-IgE antibody heavy chain coding sequence and a BsaI site were opened by BsaI and ligated through a reaction with T4 DNA ligase overnight at 37°C. The ligation reaction solution was added to *Escherichia coli* competent cells, transformed through a heat shock method, and cultured overnight at 37°C. QIAprep Spin Miniprep Kit (manufactured by QIAGEN) was used to obtain an anti-IgE antibody heavy chain coding vector from the culture medium. Similarly, potato virus X (PVX) vector pICH56131 (FIG. 3) and a synthetic DNA containing an anti-IgE antibody light chain coding sequence and a BsaI site were used to obtain an anti-IgE antibody light chain coding vector. These vectors were added to separate Agrobacterium competent cells, transformed through an electroporation method, and cultured at 28°C for 3 days. Each culture medium was mixed with one infiltration buffer (10 mM MES, 10 mM MgSO₄, pH 5.5) at 1/1000, infiltrated into *Nicotiana benthamiana* leaves, and harvested after about one week. The harvested leaves (infected leaves) were frozen.

200 g of the frozen infected leaves were weighed and pulverized with a cutter mixer. 200 mL of an extraction solution (100 mM Tris, 250 mM NaCl, and 40 mM sodium ascorbate) was added to the pulverized material, and the infected leaves were pulverized repeatedly. A supernatant was recovered through centrifugation. The recovered solution was filtered through a 0.22 µm filter. Impurities were removed by subjecting the filtrate to Protein A column chromatography.

### <Preparation of anti-IgE antibody without sugar chain-binding motif (Anti-IgE Ab-N-Q)>

An antibody whose heavy chain constant region consists of SEQ ID NO: 31 was produced by the same manner as in the antibody whose heavy chain constant region consists of SEQ ID NO: 5 (Anti-IgE Ab-Wild).

### <Preparation of anti-IgE antibody without sugar chain-binding motif (Anti-IgE Ab-T-A)>

An antibody whose heavy chain constant region sequence consists of SEQ ID NO: 44 was produced by the same manner as in the antibody whose heavy chain constant region consists of SEQ ID NO: 5 (Anti-IgE Ab-Wild).

The binding of a sugar chain to the heavy chain of Anti-IgE Ab-Wild and the absence of sugar chains bound to the respective heavy chains of anti-IgE Ab-N-Q and anti-IgE Ab-T-A were confirmed through western blotting by using an α1,3-fucose antibody.

### <Latex aggregation test of anti-IgE antibody>

### (1) Preparation of reagents

Antibodies against IgE were used to prepare measurement reagents for an immunoagglutination method as follows.
i) 0.07 mg of antibodies were added to 1 mL of a polystyrene latex suspension to prepare insoluble particles, polystyrene latex particles carrying the antibodies. These insoluble particles were suspended in a buffer solution (glycine, pH 7.3) to a concentration of 0.125 mass% to prepare an insoluble particle suspension.
ii) A buffer solution (glycine, pH 8.3) was prepared.

### (2) Measurement using automatic analyzer

A 7180 automatic analyzer manufactured by Hitachi, Ltd. was used as an automatic analyzer to measure aggregation states of insoluble particles through antigen-antibody reactions between target antigen (IgE) samples at various concentrations and the antibodies carried on the polystyrene latex particles described in (1) by light transmittance. Specifically, 140 µL of the buffer solution prepared in (1)(ii) above was added to 3.5 µL of the IgE solution, and this mixture was stirred and mixed at 37°C. After standing for 5 minutes, 70 µL of the insoluble particle suspension was added thereto and further stirred and mixed at 37°C. An aggregation reaction for about 5 minutes was measured as an amount of change in absorbance.

The insoluble particles of Anti-IgE Ab-Wild, Anti-IgE Ab-N-Q, and Anti-IgE Ab-T-A prepared in (1) above were evaluated for the degree of aggregation due to a reaction with the target antigen, and as a result, it was confirmed that the insoluble particles of Anti-IgE Ab-N-Q and Anti-IgE Ab-T-A had a higher degree of aggregation than Anti-IgE Ab-Wild, enabling highly sensitive measurement.

### <Preparation of anti-CRP antibody (Anti-CRP Ab-Wild)>

An antibody whose heavy chain constant region consists of SEQ ID NO: 1 was produced through the following method.

It was prepared in a plant transient expression system disclosed in Japanese Patent No. 5015012. Tobacco mosaic virus (TMV) vector pICH56122 (FIG. 3) and a synthetic DNA containing an anti-CRP antibody heavy chain coding sequence and a BsaI site were opened by BsaI and ligated through a reaction with T4 DNA ligase overnight at 37°C. The ligation reaction solution was added to *Escherichia coli* competent cells, transformed through a heat shock method, and cultured overnight at 37°C. QIAprep Spin Miniprep Kit (manufactured by QIAGEN) was used to obtain an anti-CRP antibody heavy chain coding vector from the culture medium. Similarly, potato virus X (PVX) vector pICH56131 (FIG. 3) and a synthetic DNA containing an anti-CRP antibody light chain coding sequence and a BsaI site were used to obtain an anti-CRP antibody light chain coding vector. These vectors were added to separate Agrobacterium competent cells, transformed through an electroporation method, and cultured at 28°C for 3 days. Each culture medium was mixed with one infiltration buffer (10 mM MES, 10 mM MgSO₄, pH 5.5) at 1/1000, infiltrated into *Nicotiana benthamiana* leaves, and harvested after about one week. The harvested leaves (infected leaves) were frozen.

200 g of the frozen infected leaves were weighed and pulverized with a cutter mixer. 200 mL of an extraction solution (100 mM Tris, 250 mM NaCl, 5 mM EDTA, and 40 mM sodium ascorbate) was added to the pulverized material, and the infected leaves were pulverized repeatedly. A supernatant was recovered through centrifugation. The recovered solution was filtered through a 0.22 µm filter. Impurities were removed by subjecting the filtrate to Protein A column chromatography.

### <Preparation of anti-CRP antibody without sugar chain-binding motif (Anti-CRP Ab-T-A)>

An antibody whose heavy chain constant region sequence consists of SEQ ID NO: 40 was produced by the same manner as in the antibody whose heavy chain constant region consists of SEQ ID NO: 1 (Anti-CRP Ab-Wild).

The binding of a sugar chain to the heavy chain of Anti-CRP Ab-Wild and the absence of a sugar chain bound to the heavy chain of anti-CRP Ab-T-A were confirmed through western blotting by using an α1,3-fucose antibody.

### <Latex aggregation test of anti-CRP antibody>

### (1) Preparation of reagents

Antibodies against CRP were used to prepare measurement reagents for an immunoagglutination method as follows.
i) 0.06 mg of antibodies were added to 1 mL of a polystyrene latex suspension to prepare insoluble particles, polystyrene latex particles carrying the antibodies. These insoluble particles were suspended in a buffer solution (glycine, pH 7.3) to a concentration of 0.110 mass% to prepare an insoluble particle suspension.
ii) A buffer solution (glycine, pH 8.3) was prepared.

### (2) Measurement using automatic analyzer

A 3500 automatic analyzer manufactured by Hitachi, Ltd. was used as an automatic analyzer to measure aggregation states of insoluble particles through antigen-antibody reactions between target antigen (CRP) samples at various concentrations and the antibodies carried on the polystyrene latex particles described in (1) by light transmittance. Specifically, 100 µL of the buffer solution prepared in (1)(ii) above was added to 2.0 µL of the CRP solution, and this mixture was stirred and mixed at 37°C. After standing for 5 minutes, 100 µL of the insoluble particle suspension was added thereto and further stirred and mixed at 37°C. An aggregation reaction for about 5 minutes was measured as an amount of change in absorbance.

The insoluble particles of Anti-CRP Ab-Wild, and Anti-CRP Ab-T-A prepared in (1) above were evaluated for the degree of aggregation due to a reaction with the target antigen, and as a result, it was confirmed that the insoluble particles of Anti-CRP Ab-T-A had a higher degree of aggregation than Anti-CRP Ab-Wild, enabling highly sensitive measurement.

The present invention is also exemplified by the following preferred embodiments:
[Item 1] An insoluble particle comprising:
   a granular carrier; and
   an antibody against a target antigen carried on the granular carrier, wherein no sugar chain is bound to a heavy chain of the antibody.
[Item 2] The insoluble particle according to item 1, wherein the granular carrier is a latex particle.
[Item 3] The insoluble particle according to item 1 or 2, wherein the antibody is IgG.
[Item 4] The insoluble particle according to item 3, wherein an Fc region of the IgG has an amino acid sequence having 90% or more identity with any one of amino acid sequences of SEQ ID NOs: 1 to 13, but does not have a sugar chain-binding motif Asn-Xaa-Ser/Thr.
[Item 5] The insoluble particle according to item 3, wherein an Fc region of the IgG has
   an amino acid sequence of SEQ ID NO: 14 or an amino acid sequence having 90% or more identity with the amino acid sequence of SEQ ID NO: 14 wherein Asp at position 180 is not mutated,
   an amino acid sequence of SEQ ID NO: 15 or an amino acid sequence having 90% or more identity with the amino acid sequence of SEQ ID NO: 15 wherein Asp at position 176 is not mutated,
   an amino acid sequence of SEQ ID NO: 16 or an amino acid sequence having 90% or more identity with the amino acid sequence of SEQ ID NO: 16 wherein Asp at position 227 is not mutated,
   an amino acid sequence of SEQ ID NO: 17 or an amino acid sequence having 90% or more identity with the amino acid sequence of SEQ ID NO: 17 wherein Asp at position 177 is not mutated,
   an amino acid sequence of SEQ ID NO: 18 or an amino acid sequence having 90% or more identity with the amino acid sequence of SEQ ID NO: 18 wherein Asp at position 174 is not mutated,
   an amino acid sequence of SEQ ID NO: 19 or an amino acid sequence having 90% or more identity with the amino acid sequence of SEQ ID NO: 19 wherein Asp at position 180 is not mutated,
   an amino acid sequence of SEQ ID NO: 20 or an amino acid sequence having 90% or more identity with the amino acid sequence of SEQ ID NO: 20 wherein Asp at position 185 is not mutated,
   an amino acid sequence of SEQ ID NO: 21 or an amino acid sequence having 90% or more identity with the amino acid sequence of SEQ ID NO: 21 wherein Asp at position 179 is not mutated,
   an amino acid sequence of SEQ ID NO: 22 or an amino acid sequence having 90% or more identity with the amino acid sequence of SEQ ID NO: 22 wherein Asp at position 176 is not mutated,
   an amino acid sequence of SEQ ID NO: 23 or an amino acid sequence having 90% or more identity with the amino acid sequence of SEQ ID NO: 23 wherein Asp at position 172 is not mutated,
   an amino acid sequence of SEQ ID NO: 24 or an amino acid sequence having 90% or more identity with the amino acid sequence of SEQ ID NO: 24 wherein Asp at position 183 is not mutated,
   an amino acid sequence of SEQ ID NO: 25 or an amino acid sequence having 90% or more identity with the amino acid sequence of SEQ ID NO: 25 wherein Asp at position 179 is not mutated, or
   an amino acid sequence of SEQ ID NO: 26 or an amino acid sequence having 90% or more identity with the amino acid sequence of SEQ ID NO: 26 wherein Asp at position 173 is not mutated.
[Item 6] The insoluble particle according to item 3,
   wherein an Fc region of the IgG has
   an amino acid sequence of SEQ ID NO: 27 or an amino acid sequence having 90% or more identity with the amino acid sequence of SEQ ID NO: 27 wherein Gln at position 180 is not mutated,
   an amino acid sequence of SEQ ID NO: 28 or an amino acid sequence having 90% or more identity with the amino acid sequence of SEQ ID NO: 28 wherein Gln at position 176 is not mutated,
   an amino acid sequence of SEQ ID NO: 29 or an amino acid sequence having 90% or more identity with the amino acid sequence of SEQ ID NO: 29 wherein Gln at position 227 is not mutated,
   an amino acid sequence of SEQ ID NO: 30 or an amino acid sequence having 90% or more identity with the amino acid sequence of SEQ ID NO: 30 wherein Gln at position 177 is not mutated,
   an amino acid sequence of SEQ ID NO: 31 or an amino acid sequence having 90% or more identity with the amino acid sequence of SEQ ID NO: 31 wherein Gln at position 174 is not mutated,
   an amino acid sequence of SEQ ID NO: 32 or an amino acid sequence having 90% or more identity with the amino acid sequence of SEQ ID NO: 32 wherein Gln at position 180 is not mutated,
   an amino acid sequence of SEQ ID NO: 33 or an amino acid sequence having 90% or more identity with the amino acid sequence of SEQ ID NO: 33 wherein Gln at position 185 is not mutated,
   an amino acid sequence of SEQ ID NO: 34 or an amino acid sequence having 90% or more identity with the amino acid sequence of SEQ ID NO: 34 wherein Gln at position 179 is not mutated,
   an amino acid sequence of SEQ ID NO: 35 or an amino acid sequence having 90% or more identity with the amino acid sequence of SEQ ID NO: 35 wherein Gln at position 176 is not mutated,
   an amino acid sequence of SEQ ID NO: 36 or an amino acid sequence having 90% or more identity with the amino acid sequence of SEQ ID NO: 36 wherein Gln at position 172 is not mutated,
   an amino acid sequence of SEQ ID NO: 37 or an amino acid sequence having 90% or more identity with the amino acid sequence of SEQ ID NO: 37 wherein Gln at position 183 is not mutated,
   an amino acid sequence of SEQ ID NO: 38 or an amino acid sequence having 90% or more identity with the amino acid sequence of SEQ ID NO: 38 wherein Gln at position 179 is not mutated, or
   an amino acid sequence of SEQ ID NO: 39 or an amino acid sequence having 90% or more identity with the amino acid sequence of SEQ ID NO: 39 wherein Gln at position 173 is not mutated.
[Item 7] The insoluble particle according to item 3,
   wherein an Fc region of the IgG has
   an amino acid sequence of SEQ ID NO: 40 or an amino acid sequence having 90% or more identity with the amino acid sequence of SEQ ID NO: 40 wherein Ala at position 182 is not mutated,
   an amino acid sequence of SEQ ID NO: 41 or an amino acid sequence having 90% or more identity with the amino acid sequence of SEQ ID NO: 41 wherein Ala at position 178 is not mutated,
   an amino acid sequence of SEQ ID NO: 42 or an amino acid sequence having 90% or more identity with the amino acid sequence of SEQ ID NO: 42 wherein Ala at position 229 is not mutated,
   an amino acid sequence of SEQ ID NO: 43 or an amino acid sequence having 90% or more identity with the amino acid sequence of SEQ ID NO: 43 wherein Ala at position 179 is not mutated,
   an amino acid sequence of SEQ ID NO: 44 or an amino acid sequence having 90% or more identity with the amino acid sequence of SEQ ID NO: 44 wherein Ala at position 176 is not mutated,
   an amino acid sequence of SEQ ID NO: 45 or an amino acid sequence having 90% or more identity with the amino acid sequence of SEQ ID NO: 45 wherein Ala at position 182 is not mutated,
   an amino acid sequence of SEQ ID NO: 46 or an amino acid sequence having 90% or more identity with the amino acid sequence of SEQ ID NO: 46 wherein Ala at position 187 is not mutated,
   an amino acid sequence of SEQ ID NO: 47 or an amino acid sequence having 90% or more identity with the amino acid sequence of SEQ ID NO: 47 wherein Ala at position 181 is not mutated,
   an amino acid sequence of SEQ ID NO: 48 or an amino acid sequence having 90% or more identity with the amino acid sequence of SEQ ID NO: 48 wherein Ala at position 178 is not mutated,
   an amino acid sequence of SEQ ID NO: 49 or an amino acid sequence having 90% or more identity with the amino acid sequence of SEQ ID NO: 49 wherein Ala at position 174 is not mutated,
   an amino acid sequence of SEQ ID NO: 50 or an amino acid sequence having 90% or more identity with the amino acid sequence of SEQ ID NO: 50 wherein Ala at position 185 is not mutated,
   an amino acid sequence of SEQ ID NO: 51 or an amino acid sequence having 90% or more identity with the amino acid sequence of SEQ ID NO: 51 wherein Ala at position 181 is not mutated, or
   an amino acid sequence of SEQ ID NO: 52 or an amino acid sequence having 90% or more identity with the amino acid sequence of SEQ ID NO: 52 wherein Ala at position 175 is not mutated.
[Item 8] A kit for measuring a target antigen, comprising:
   the insoluble particle according to any one of items 1 to 7; or
   an antibody or a granular carrier for preparing the insoluble particle according to any one of items 1 to 7.
[Item 9] The kit according to item 8 for qualitatively evaluating the presence or absence of the target antigen in a test sample or for quantitatively evaluating a concentration of the target antigen in a test sample.
[Item 10] A method for measuring a target antigen, wherein the insoluble particle according to any one of items 1 to 7 is used.
[Item 11] A method for measuring a target antigen, comprising:
   a step of bringing the insoluble particle according to any one of items 1 to 7 into contact with a test sample that may contain the target antigen; and
   a step of measuring an aggregation reaction of the insoluble particles due to an antigen-antibody reaction between the antibody and the target antigen.
[Item 12] The method according to item 10 or **11** for qualitatively evaluating the presence or absence of the target antigen in a test sample or for quantitatively evaluating a concentration of the target antigen in a test sample.

## Claims

1. A method for measuring a target antigen, comprising:
a step of bringing an insoluble particle into contact with a test sample that may contain the target antigen; and
a step of measuring an aggregation reaction of the insoluble particles due to an antigen-antibody reaction between an antibody and the target antigen, wherein
the insoluble particle comprises a latex particle and the antibody against the target antigen carried on the latex particle;
no sugar chain is bound to a heavy chain of the antibody; and
the antibody is IgG.

2. The method according to claim 1, wherein an Fc region of the IgG has an amino acid sequence having 90% or more identity with any one of amino acid sequences of SEQ ID NOs: 5 to 8, but does not have a sugar chain-binding motif Asn-Xaa-Ser/Thr.

3. The method according to claim 1, wherein an Fc region of the IgG has
an amino acid sequence of SEQ ID NO: 18 or an amino acid sequence having 90% or more identity with the amino acid sequence of SEQ ID NO: 18 wherein Asp at position 174 is not mutated,
an amino acid sequence of SEQ ID NO: 19 or an amino acid sequence having 90% or more identity with the amino acid sequence of SEQ ID NO: 19 wherein Asp at position 180 is not mutated,
an amino acid sequence of SEQ ID NO: 20 or an amino acid sequence having 90% or more identity with the amino acid sequence of SEQ ID NO: 20 wherein Asp at position 185 is not mutated, or
an amino acid sequence of SEQ ID NO: 21 or an amino acid sequence having 90% or more identity with the amino acid sequence of SEQ ID NO: 21 wherein Asp at position 179 is not mutated.

4. The method according to claim 1,
wherein an Fc region of the IgG has
an amino acid sequence of SEQ ID NO: 31 or an amino acid sequence having 90% or more identity with the amino acid sequence of SEQ ID NO: 31 wherein Gln at position 174 is not mutated,
an amino acid sequence of SEQ ID NO: 32 or an amino acid sequence having 90% or more identity with the amino acid sequence of SEQ ID NO: 32 wherein Gln at position 180 is not mutated,
an amino acid sequence of SEQ ID NO: 33 or an amino acid sequence having 90% or more identity with the amino acid sequence of SEQ ID NO: 33 wherein Gln at position 185 is not mutated, or
an amino acid sequence of SEQ ID NO: 34 or an amino acid sequence having 90% or more identity with the amino acid sequence of SEQ ID NO: 34 wherein Gln at position 179 is not mutated.

5. The method according to claim 1, wherein an Fc region of the IgG has
an amino acid sequence of SEQ ID NO: 44 or an amino acid sequence having 90% or more identity with the amino acid sequence of SEQ ID NO: 44 wherein Ala at position 176 is not mutated,
an amino acid sequence of SEQ ID NO: 45 or an amino acid sequence having 90% or more identity with the amino acid sequence of SEQ ID NO: 45 wherein Ala at position 182 is not mutated,
an amino acid sequence of SEQ ID NO: 46 or an amino acid sequence having 90% or more identity with the amino acid sequence of SEQ ID NO: 46 wherein Ala at position 187 is not mutated, or
an amino acid sequence of SEQ ID NO: 47 or an amino acid sequence having 90% or more identity with the amino acid sequence of SEQ ID NO: 47 wherein Ala at position 181 is not mutated.

6. The method according to any one of claims 1 to 5 for qualitatively evaluating the presence or absence of the target antigen in a test sample or for quantitatively evaluating a concentration of the target antigen in a test sample.
